# EUROPEAN PATENT SPECIFICATION

(11) **EP 3 052 528 B1**
(45) Date of publication and mention of the grant of the patent: **22.11.2017**
(21) Application number: 13773696.3
(22) Date of filing: 02.10.2013
(51) Int. Cl.: C08B 37/08, A61L 27/52, C08J 3/075, C08J 3/24, C08L 5/08

(54) **THERMOSENSITIVE HYALURONIC ACID CONJUGATES AND METHODS FOR THE PREPARATION THEREOF**
TEMPERATUREMPFINDLICHE HYALURONSÄUREKONJUGATE UND DEREN HERSTELLUNG
CONJUGUES D'ACIDE HYALURONIQUE THERMOSENSIBLES ET METHODES DE PREPARATION

(43) Date of publication of application: 10.08.2016
(73) Proprietor: AO Technology AG, 7000 Chur (CH)
(72) Inventor: D'ESTE, Matteo, CH-7270 Davos Platz (CH); EGLIN, David Olivier, CH-7276 Davos Frauenkirch (CH); RICHARDS, Robert Geoffrey, CH-7260 Davos Dorf (CH); ALINI, Mauro, CH-7270 Davos Platz (CH)
(74) Representative: Schmitz, Joseph
(86) International application number: PCT/EP2013/070519
(87) International publication number: WO 2015/048988

(56) References cited:
- WO-A1-2010/099818
- BERGMAN K ET AL: "Hyaluronic acid derivatives prepared in aqueous media by triazine-activated amidation", BIOMACROMOLECULES, AMERICAN CHEMICAL SOCIETY, US, vol. 8, no. 7, 1 July 2007 (2007-07-01), pages 2190-2195, XP002566505, ISSN: 1525-7797, DOI: 10.1021/BM0701604 [retrieved on 2007-06-19] cited in the application
- MATTEO D'ESTE ET AL: "Single step synthesis and characterization of thermoresponsive hyaluronan hydrogels", CARBOHYDRATE POLYMERS, vol. 90, no. 3, 1 October 2012 (2012-10-01), pages 1378-1385, XP055088400, ISSN: 0144-8617, DOI: 10.1016/j.carbpol.2012.07.007 cited in the application
- DEREK MORTISEN ET AL: "Tailoring Thermoreversible Hyaluronan Hydrogels by "Click" Chemistry and RAFT Polymerization for Cell and Drug Therapy", BIOMACROMOLECULES, vol. 11, no. 5, 10 May 2010 (2010-05-10), pages 1261-1272, XP055088419, ISSN: 1525-7797, DOI: 10.1021/bm100046n
- MARIANNA PEROGLIO ET AL: "Injectable thermoreversible hyaluronan-based hydrogels for nucleus pulposus cell encapsulation", EUROPEAN SPINE JOURNAL, SPRINGER, BERLIN, DE, vol. 21, no. 6, 27 August 2011 (2011-08-27), pages 839-849, XP035093666, ISSN: 1432-0932, DOI: 10.1007/S00586-011-1976-2
- N Horn ET AL: "Antibacterial Activity of Antibiotic loaded Thermo-responsive Hyaluronan Hydrogel", European Cells and Materials, 1 January 2011 (2011-01-01), page 54, XP055088572, Retrieved from the Internet: URL:http://www.ecmjournal.org/journal/supp lements/vol021supp02/pdf/v021supp02a54.pdf [retrieved on 2013-11-15]
- TAN H ET AL: "Thermosensitive injectable hyaluronic acid hydrogel for adipose tissue engineering", BIOMATERIALS, ELSEVIER SCIENCE PUBLISHERS BV., BARKING, GB, vol. 30, no. 36, 1 December 2009 (2009-12-01), pages 6844-6853, XP026708776, ISSN: 0142-9612, DOI: 10.1016/J.BIOMATERIALS.2009.08.058 [retrieved on 2009-09-26]

## Description

### TECHNICAL FIELD

The present invention relates to the thermosensitive hyaluronic acid conjugates and methods for the preparation thereof.

### PRIOR ART

| | |
|---|---|
| WO 2010/099818 | (AO Technology AG) |
| EP0216453 | (Fidia S.p.a) |
| EP1095064 | (Fidia Farmaceutici S.p.a) |
| CN102772823 | (South China University) |

Recent development of peptide coupling reagents in organic synthesis - (Tetrahedron 60, 2004, 2447-2467)
Recent applications of 2,4,6-trichloro-1,3,5-triazine and its derivatives in organic synthesis *-* (Tetrahedron 62, 2006, 9507-9522)
Efficient activation of carboxyl polysachharides for preparation of conjugates - (Carbohydrate Polymers 668, 2007, 187-190)
Diels-Alder "Click" crosslinked hyaluronic acid hydrogels for tissue engineering - (Biomacromolecules, 2011, 12, 824-830*)*
Hyaluronic acid derivatives prepared in aqueous media by triazine-activated amidation - (Biomacromolecules, 2007, 8, 2190-2195*)*
Single step synthesis and characterization of thermoresponsive hyaluronan hydrogels (Carbohydrate Polymers, 90, 2012, 1378-1385)
Preparation of thermo-responsive and injectable hydrogels based on hyaluronic acid and poly(N-isopropylacrylamide) and their Drug release behaviours (Macromolecular Research, Vol.14, No.1, pp 87 - 93, 2006)
Biological evaluation of tissue-engineered cartilage using thermoresponsive poly(N-isopropylacrylamide)-grafted hyaluronan (Journal of Biomaterials and Nanobiotechnology, 2012, 3, 1-9*)*

### BACKGROUND OF THE INVENTION

Hyaluronic acid (HA), or hyaluronate, is an unbranched, naturally occurring biopolymer consisting of alternating residues of D-glucuronic acid and N-acetyl-D-glucosamine, and is one of the chief components of the extracellular matrix.

Hyaluronic acid has a molecular weight ranging of from 50 kDa to 10 000 kDa, and can be prepared via extraction from tissue or fermentation of certain microorganisms, if necessary according to rigorous specifications when used as medical implantable material.

Hyaluronic acid can be, and has been, chemically or enzymatically modified in order to produce semi-synthetic derivatives with tailored rheological, physico-chemical and biological properties.

Hyaluronic acid is ubiquitous, non-immunogenic, and a chief component of the extracellular matrix of various connective tissues with key functions in wound healing and the regeneration of tissue.

Owing to the above-mentioned features, hyaluronic acid has been employed since over 30 years in different biomedical applications including ophthalmic surgery, osteoarthritis treatment, wound management, space filler for aesthetic surgery or tissue augmentation, adhesion barrier, productions of scaffolds and hydrogels for tissue engineering.

The use of hyaluronic acid in research is even broader. Hyaluronic acid is an extremely hydrophilic molecule with marked tendency to fill the surrounding space. This is the reason why hyaluronic acid fills the extracellular matrix of many connective tissues. Interestingly, hyaluronic acid is known to modulate cell migration, and even more interestingly it is also known to target actively cell-surface receptors such as CD44, CD54 (also known as ICAM-1) and CD168 (also known as RHAMM). Interaction with CD44, which is expressed by many solid tumors, is exploited for the active targeting of antineoplastic drugs. Thus, applications for hyaluronic acid that are currently being researched include its use for the preparation of drug delivery systems and stimulus-responsive hydrogels.

Even though extremely hydrophilic in its native form, the water solubility of hyaluronic acid can be modulated via chemical modification. Such derivatives are extremely interesting because they display different physico-chemical, rheological, degradation, host tissue response properties, but are still recognized as hyaluronic acid by cells in spite of the chemical modification.

Examples of such derivatives include hyaluronic acid esters (disclosed in EP 216453), hyaluronic acid amides (disclosed in EP 1095064) and hyaluronic acid based artificial extracellular matrices (Adv Mater. 2011 Mar 25;23(12):H41-56).

As regards modifications of hyaluronic acid, a further advancement is the possibility of reversibly modulating the solubility of hyaluronic acid by applying one or more external stimuli. Such "stimulus-responsive" materials can be obtained by grafting molecules endowed with this responsiveness to hyaluronic acid (Carbohydrate Polymers 90 (2012) 1378-1385, Biomacromolecules 2010, 11, 1261-1272, WO2010099818).

A remarkable example of "stimulus-responsive" materials are thermoresponsive polymers (TPs). The chemical structure of thermoresponive polymers features different functional groups able to establish hydrogen bonding, but also hydrophobic interactions, and the thermodynamic balance between these modalities of bonding is temperature-dependent. As a result, thermoresponsive polymers are swollen and fully hydrated at temperatures below their lower critical solution temperature (LCST), but shrunken and insoluble at temperatures above their lower critical solution temperature (LCST).

In the field of biomedical applications, a prominent thermoresponsive polymer is poly(N-isopropylacrylamide) (pNIPAM), which features a sharp transition between solvation and gelation in water at a lower critical solution temperature (LCST) of 32°C, which is a physiologically relevant point between room and body temperature (Carbohydrate Polymers 90 (2012) 1378- 1385, Biomacromolecules 2010, 11, 1261-1272, WO2010099818).

In WO2010099818, a thermosensitive hyaluronic acid conjugate of hyaluronic acid and pNIPAM is disclosed, as well as a method of preparing such conjugates by "click-chemistry". When heated to a temperature above their lower critical solution temperature (LCST), these conjugates give rise to a hydrogel structure. Since the molecular interactions within the formed hydrogel are of non-covalent nature, the transition is reversible and can be brought about by decreasing the temperature below the lower critical solution temperature (LCST), and the conjugate becomes a free flowing solution again.

Other suitable methods for the preparation of a "stimulus-responsive" material based on hyaluronic acid have been described in Carbohydrate Polymers 90 (2012) 1378- 1385, where a thermosensitive hyaluronic acid conjugate of hyaluronic acid and pNIPAM or JEFFAMINES® is obtained through direct amidation by 1,1'-carbonyldiimidazole activation. While the method according to the above publication yields satisfactory degrees of substitution, the preparation of such conjugates according to the above method requires the tedious conversion of the commercially available sodium salt of hyaluronic acid into the corresponding tertabutylammonium salt via cationic exchange before the actual amidation can take place, and must be carried out in an organic solvent, namely DMSO, which must be eliminated by dialysis after the amidation reaction finishes. The obtained conjugates exhibit acceptable viscosities below and above their LCST, even though a higher viscosity below LCST would be highly desirable for example in biomedical applications where an injected conjugate should not flow away (known as "bleeding") from the site of injection before the corresponding stimulus can induce gelling.

There exists thus a need to provide novel "stimulus-responsive" materials based on hyaluronic acid, especially thermosensitive materials, whose method of preparation is less laborious and does not require the use of organic solvents. Furthermore, it is desirable to provide for a method of preparation of thermosensitive materials exhibiting enhanced rheological properties when compared to available thermosensitive materials, especially materials with reduced bleed and improved thermosensitive rheological transition.

### SUMMARY OF THE INVENTION

The present invention provides for a method for preparing a thermosensitive hyaluronic acid conjugate comprising the steps of contacting, in any order, preferably in this order, a. a predefined amount of hyaluronic acid, b. a predefined amount of a 1,3,5-triazine (or s-triazine) compound and c. a predefined amount of one or more thermosensitive polymers having at least one terminal amine moiety.

The present invention further provides for a thermosensitive hyaluronic acid conjugate obtained by a method comprising the steps of contacting, in any order, preferably in this order, a. a predefined amount of hyaluronic acid, b. a predefined amount of a 1,3,5-triazine (or s-triazine) compound and c. a predefined amount of one or more thermosensitive polymers having at least one terminal amine moiety.

The present invention further provides for a bioactive agent delivery system comprising the thermosensitive hyaluronic acid conjugate thus and one or more bioactive agents.

Further embodiments of the invention are laid down in the dependent claims.

In the context of the present invention, the term "degree of substitution" refers to the percentage of available carboxylic acid moieties of a predetermined amount of hyaluronic acid that have reacted with a thermosensitive polymer having at least one terminal amine moiety, when measured using ¹H nuclear magnetic resonance spectroscopy according to the procedure in Carbohydrate Polymers 90 (2012) 1378-1385.

In the context of the present invention, the terms "thermoresponsive" and "thermosensitive" are used interchangeably and refer to the ability of a material to undergo a change in physical properties depending on its temperature.

In the context of the present invention, the term "hyaluronic acid" and "hyaluronan" are used interchangeably and refer to both hyaluronic acid in acidic form and its salts of mono-or divalent cations, preferably monovalent cations, and among them preferably alkaline metals cations, the most preferred being sodium. Therefore, within the present document the words "hyaluronic acid" or "hyaluronan" are used as synonyms, indicating the polymer in its dissociated or undissociated form.

In the context of the present invention, the term " a solution essentially free of organic solvents" refers to a solution comprising less than 1 weight percent, and preferably less than 0.1 weight percent of organic solvents.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

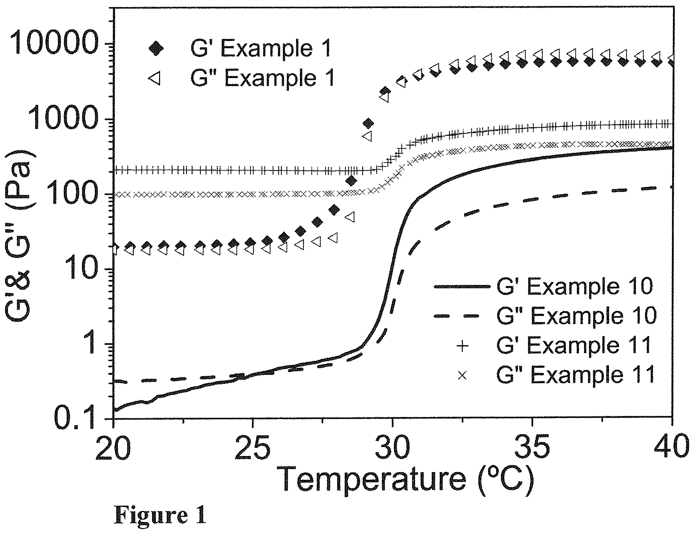

- Fig. 1: shows a the rheological profiles (shear storage modulus G' and shear loss modulus G") of thermosensitive hyaluronic acid conjugates prepared from hyaluronic acid and pNIPAM using different compounds such as DMTMM (inventive, according to Example 1), CDI (comparative, according to Example 10) and EDC/NHS (comparative, according to Example 11), across a temperature range of from 20 to 40°C.

### DESCRIPTION OF PREFERRED EMBODIMENTS

The present invention provides for a method for preparing a thermosensitive hyaluronic acid conjugate comprising the steps of contacting, in any order, preferably in this order, a. a predefined amount of hyaluronic acid, b. a predefined amount of a 1,3,5-triazine (or s-triazine) compound and c. a predefined amount of one or more thermosensitive polymers having at least one terminal amine moiety.

As used herein the term "thermosensitive polymer" is used interchangeably with "thermoresponsive polymer" and refers to a polymer capable of forming a hydration shell in an aqueous solution when at a temperature below its lower critical solution temperature (LCST) and capable to undergo significant changes in hydration when brought to a temperature above its lower critical solution temperature (LCST).

The one or more thermosensitive polymers having at least one terminal amine moiety may be selected from the group polyacrylamides, polyvinylamides, polyalkyloxazolines, polyvinylimidazoles, poloxamers, polyetheramines, peptide amphiphiles, and their copolymers and blends.

Specific examples for such thermosensitive polymers having at least one terminal amine moiety are poly (N- isopropylacrylamide) (pNIPAM), poly (N-vinylcaprolactam), poly(N-isopropyloxazoline), poly (vinylimidazole) and poly (N- acryloyl-pyrrolidin), poly(N-alkyl(meth)acrylamide)s, polyetheramines and poloxamers.

As used herein the term "poly(N-alkyl(meth)acrylamide)" refers to both poly(N-alkylmethacrylamide) or poly(N-alkylacrylamide).

In a preferred embodiment, a predefined amount of one or more thermosensitive polymer, preferably solubilized in an aqueous solvent, is contacted with a predefined amount of hyaluronic acid preferably solubilized in an aqueous solvent, and a predefined amount of a 1,3,5-triazine (or s-triazine) compound is subsequently contacted with the mixture of solubilized one or more thermosensitive polymer and hyaluronic acid.

In an alternative embodiment, a predefined amount of hyaluronic acid, preferably solubilized in an aqueous solvent, is contacted with a predefined amount of a 1,3,5-triazine (or s-triazine) compound, and the predefined amount of one or more thermosensitive polymer, preferably solubilized in an aqueous solvent, is subsequently contacted with the mixture of solubilized hyaluronic acid and 1,3,5-triazine.

In another alternative embodiment, a predefined amount of one or more thermosensitive polymer, preferably solubilized in an aqueous solvent, is contacted with a predefined amount of a 1,3,5-triazine (or s-triazine) compound, and a predefined amount of hyaluronic acid, preferably solubilized in an aqueous solvent, is subsequently contacted with the mixture of solubilized one or more thermosensitive polymer and 1,3,5-triazine.

In a preferred embodiment of the method, the hyaluronic acid and the thermosensitive polymer are jointly solubilized in an aqueous solvent, preferably in an aqueous buffer solution having a pH of from 4.0 to 9.5, preferably between 4.5 and 7.5, and even more preferably between 5.0 and 7.5 or 5.0 to 6. Most preferably the aqueous solvent or aqueous buffer solution is essentially free of organic solvents.

The predetermined amount of hyaluronic acid may be dissolved in an aqueous solvent such as water or aqueous buffer solution such as to realize a concentration of hyaluronic acid from 0.1 to 15% w/v, preferably of from 0.1 to 5% w/v, and in particular around 0.7% w/v, 1.0% w/v or 1.5% w/v depending on the molecular weight and the desired viscosity of the obtained solution. In general, a high molecular weight hyaluronic acid will yield a higher viscosity at a given w/v concentration than a low molecular weight hyaluronic acid.

The aqueous solvent suitable for the solubilisation of the hyaluronic acid and/or the thermosensitive polymer may be any of the commonly used aqueous solvents well known by those of ordinary skill in the art. The aqueous solvent may be selected from the group consisting of water, pure or ultrapure water (including water for injections), aqueous buffer solutions, acid solutions, basic solutions, salt solutions, saline solution, and glucose salt solution. Aqueous buffer solutions having a pH of from 4.0 to 9.5 are for example sodium acetate buffer, phosphate buffer saline, Tris buffer, sodium phosphate buffer, MOPS, PIPES, MES and potassium phosphate (e.g. in the range of 25 mM to 500 mM and in the pH range of 4.0 to 9.5). The aqueous solvent or aqueous buffer solution can also contain up to 60% of organic solvents. Examples of organic solvents include but are not limited to alcohols, DMF, DMSO, NMP, Acetonitrile, Ethanol, Methanol, Propanol (n- or iso-), butanol, dioxane, THF. Preferably the aqueous solvent or aqueous buffer solution are free of organic solvents, meaning that the organic solvent content of the aqueous solvent or aqueous buffer solution does not exceed 0.1% by weight, based on the total weight of the aqueous solvent or aqueous buffer solution.

The method according to the present invention may proceed for up to one week at a temperature between + 2 and + 50 °C, preferably at a temperature between + 2 and +8 °C. In the case where the method according to the present invention is performed in an aqueous solvent free of organic solvent such as for example water, aqueous saline or buffer solution, the method is carried out preferably below room temperature, specifically between + 2 and + 15 °C, yet more preferably between + 2 and 8 °C. More specifically, examples of the reaction duration include but are not limited to 4h, 8h, 12h, 16h, 20h, 24h, 2 days, 3 days, 5 days and 7 days. In a preferred embodiment the reaction is left to proceed for 3 to 5 days.

The final product is purified and isolated according to standard Organic Chemistry and Polymer Chemistry procedures well known to the person with ordinary skills in the field, e.g. via dialysis, freeze drying, extraction, crystallization, precipitation induced by solvents addition or evaporation, precipitation or gelling induced by temperature change or their combination.

In another embodiment of the method, the thermosensitive polymer having at least one terminal amine moiety is chosen among poly(N-alkyl(meth)acrylamide)s, polyetheramines or poloxamers, and more preferably the thermosensitive polymer having at least one terminal amine moiety is a poly(N-alkyl(meth)acrylamide), more preferably poly(N-isopropylacrylamide).

Poloxamers suitable in the present invention are copolymers of ethylene oxide and propylene oxide comprising a central chain of poly(propylene oxide) flanked by chains of poly(ethylene oxide) and comprising at least one terminal amine moiety.
Poloxamers useful in the present invention can be obtained by modifying poloxamers of the general formula HO(C₂H₄O)ₐ(C₃H₆O)_{b}(C₂H₄O)ₐH such as to introduce a terminal amine moiety by methods known in the art, where for example a:b = 12:20; 80:27; 64:37; 141:44; 101:56; or other examples of poloxamer included in the PhEur 6.0 and USP32-NF27. Poloxamers are commercially available under trademarks such as Synperonics, Pluronics, Kolliphor, Lutrol; Monolan; Pluronic; poloxalkol; poloxamera or Supronic.

Polyetheramines suitable in the present invention are commercially available under the trademark JEFFAMINES®, and comprise primary amino groups attached to the end of a polyether backbone. The polyether backbone is normally based on either propylene oxide, ethylene oxide or a mixture of propylene and ethylene oxide with a ratio of propylene oxide to ethylene oxide monomers ranging from 20 to 0.05 and preferably from 10 to 0.15. Suitable polyetheramines can be chosen among mono-, di-, or triamine polyetheramines, and preferably the polyetheramine is a monoamine polyetheramine. Further included as suitable polyetheramines are secondary, hindered, high-conversion, and polytetramethylene glycol based polyetheramines.

In a specific embodiment where the thermosensitive polymer is a poly(N-alkyl(meth)acrylamide) and in particular pNIPAM, the hyaluronic acid and the poly(N-alkyl(meth)acrylamide) are contacted and solubilized in pure water and the 1,3,5-triazine, and in particular a salt of 4-(4,6-dialkyloxy-1,3,5-triazin-2-yl)-4-methyl morpholinium, is subsequently added to the solution of hyaluronic acid and poly(N-alkyl(meth)acrylamide). The pH of the thus obtained mixture can be adjusted to a value of from 5 to 7.5, and may be kept within said range throughout the reaction duration, by addition of an acidic or an alkaline compound such as for example aqueous solutions of hydrochloric acid (HCl) having a molarity of from 0.01 to 10 M, such as for example 0.1 M, 1 M, 10 M, or aqueous solutions of sodium hydroxide (NaOH) having a molarity of from 0.01 to 10 M, such as for example 0.1 M, 1 M, 10 M.

In another specific embodiment where the one or more thermosensitive polymer is a polyetheramine or a poloxamer (such as commercially available JEFFAMINE® or PLURONIC®) are contacted and solubilized in buffer-less, pure water and the 1,3,5-triazine and in particular a salt of 4-(4,6-dialkyloxy-1,3,5-triazin-2-yl)-4-methyl morpholinium is subsequently added to the solution of hyaluronic acid and poly(N-alkyl(meth)acrylamide). The pH of the thus obtained mixture can be adjusted to a value of from 4.0 and 9.5, preferably between 4.5 and 7.5, and even more preferably between 5.0 and 7.5, and may be kept within said range throughout the reaction duration, by addition of an acidic or an alkaline compound, such as for example aqueous solutions of hydrochloric acid (HCl) having a molarity of from 0.01 to 10 M, such as for example 0.1 M, 1 M, 10 M, or aqueous solutions of sodium hydroxide (NaOH) having a molarity of from 0.01 to 10 M, such as for example 0.1 M, 1 M, 10 M.

In a further preferred embodiment of the method, the hyaluronic acid has a molecular weight of from 50 to 10 000 kDa, preferably 50 to 2 500 kDa and more preferably of from 100 to 1000 kDa. Sources of hyaluronic acid include extraction from tissue or by biotech techniques such as fermentation of genetically modified B. subtilis.

In yet another embodiment of the method, the 1,3,5-triazine (or s-triazine) compound is a salt of 4-(4,6-dialkyloxy-1,3,5-triazin-2-yl)-4-methyl morpholinium or 4-(4,6-dihalogeno-1,3,5-triazin-2-yl)-4-methyl morpholinium and is more preferably is 4-(4,6-dimethoxy-1,3,5-triazin-2-yl)-4-methyl morpholinium chloride or 4-(4,6-dimethoxy-1,3,5-triazin-2-yl)-4-methyl morpholinium tetrafluoroborate.

In another embodiment of the method, the predetermined amount of hyaluronic acid and of the 1,3,5-triazine compound are chosen such that the molar ratio between the available carboxylic acid moieties of the hyaluronic acid, or D-glucuronic acid monomers, and the 1,3,5-triazine (or s-triazine) compound is of from 0.05 to 20, preferably of from 0.5 to 10, more preferably from 0.8 to 4, and most preferably of from 1 to 2.

In an additional embodiment of the method, the one or more thermosensitive polymers having at least one terminal amine moiety have a molecular weight of from 5 to 200 kDa, preferably of from 10 to 100 kDa. In the case where the one or more thermosensitive polymer is a poly(N-alkyl(meth)acrylamide), the molecular weight of the thermosensitive polymer is preferably of from 15 to 50 kDa.

In one embodiment the thermosensitive hyaluronic acid conjugate is isolated from the reaction mixture via precipitation through the slow addition of ethanol or methanol after adjustment of the ionic strength with electrolytes to a ionic strength typically required for precipitation of hyaluronic and its derivatives. In an alternative embodiment the product is isolated via crystallization. In another embodiment the product is isolated via solvent evaporation. In yet another embodiment the product is dialyzed against water or salt solutions and freeze dried to obtain a dry lyophilized.

In a preferred embodiment the thermosensitive hyaluronic acid conjugate can be isolated by increasing the temperature of the reaction mixture above the lower critical solution temperature (LCST), e.g. at 40°C, 50°C or 60°C, and subsequent washing with hot water until residual waste products are eliminated, before finally being freeze-dried for storage. The lower critical solution temperature (LCST) of the conjugate can be derived from the thermosensitive polymer that is covalently linked to the hyaluronic acid.

The present invention further provides for a thermosensitive hyaluronic acid conjugate obtained by the method described above.

The present invention further provides for a solution of the thermosensitive hyaluronic acid conjugate, comprising said thermosensitive hyaluronic acid conjugate in an amount of from 1 to 95% w/v, 1 to 50% w/v, 1 to 20 w/v% or 5 to 15w/v in an aqueous or aqueous buffer solution, wherein the aqueous solvent or aqueous buffer solution is preferably essentially free of organic solvent.

Thus, the present invention also provides for a solution of the thermosensitive hyaluronic acid conjugate in an aqueous solvent or aqueous buffer solution, said solution having a shear storage modulus G' in excess of 8 Pa below its LCST, preferably of from 8 to 100 Pa below its LCST and a ratio between said storage modulus G' above its LCST and said modulus G' below its LCST, i.e. G'(>LCST)/G'(<LCST), in excess of 80, preferably of from 80 to 8000, more preferably of from 80 to 4000, most preferably of from 100 to 3000. Stated alternatively, the solutions of the thermosensitive hyaluronic acid conjugates of the present invention are able stiffen more than 80-fold when heated above their LCST.

Thus, the present invention also provides for a solution of the thermosensitive hyaluronic acid conjugate in an aqueous solvent or aqueous buffer solution, said solution having a shear storage modulus G' in excess of 8 Pa at 25°C, preferably of from 8 to 100 Pa at 25°C, and a ratio between their storage modulus at 35°C and 25°C, i.e. G'(35°C)/G'(25°C) is in excess of 80, preferably of from 80 to 8000, more preferably of from 80 to 4000, most preferably of from 100 to 3000.

The conjugation of hyaluronic acid with one or more thermosensitive polymers having at least one terminal amine moiety via a direct amidation reaction mediated by 1,3,5-triazine (or s-triazine) compound surprisingly yields a product with improved viscoelasticity profile and therefore improved performance for medical applications, in contrast to products obtained via direct or indirect amidation reaction mediated by carbonyl diimidazole or carbodiimide derivatives.

The thermosensitive hyaluronic acid conjugates obtained according to the method disclosed in the present invention display temperature-dependent solubility in water. They give rise to clear and/or flowable solutions at room temperature, which turn into a hydrocolloid gel state upon heating above their respective LCST. This transition is accompanied by a change in the rheological properties of the thermosensitive hyaluronic acid conjugates. Surprisingly, the thermosensitive hyaluronic acid conjugates obtained according to the method disclosed exhibit systematically improved viscoelasticity profile when compared to hyaluronic acid conjugates obtained through known methods.

The present invention further provides for a thermosensitive hyaluronic acid conjugate obtained by the method provided above having a degree of substitution of from 0.1 to 50%, preferably of from 2 to 30%, more preferably of from 2.5 to 25% and most preferably of from 2.5 to 10% when measured by nuclear magnetic resonance spectroscopy. In the case where the one or more thermosensitive polymer is a poly(N-alkyl(meth)acrylamide), the degree of substitution is preferably of from 2.5 to 10% when measured by nuclear magnetic resonance spectroscopy.

The thermosensitive hyaluronic acid conjugate according to the present invention may also be suitable for use in the treatment of internal or external body trauma, in a drug delivery system, in an antibiotic delivery system or other an anti-infective delivery systems (including but not limited to additives such as, disinfectants, antimicrobial peptides, quorum sensing inhibitors to prevent biofilm formation, silver (in any pharmaceutical form), nanoparticulates, anti-bacterial adhesins to prevent bacterial attachment, anti MSCRAMMs (microbial surface components recognizing adhesive matrix molecules)) or in the treatment of bone or cartilage defects.

The present invention further provides for a bioactive agent delivery system comprising the thermosensitive hyaluronic acid conjugate obtainable by the above-mentioned method and one or more bioactive agent.

The one or more bioactive agent may be selected from inorganic salts to a macromolecular compound or a small molecule compound, such as strontium salts, strontium ranelates, bisphosphonates, etidronates, clodronates tiludronates, pamidronates, neridronates, olpadronates, alendronates, ibandronates, risedronates zoledronates, icaritin and analogues, kartogenin and analogues, peptides, proteins, oligo- and poly-nucleotides, antibiotics, antibacterials, antimicrobics, disinfectants, antiseptics, bactericidal and bacteriostatic substances for infection prevention and infection treatment such as aminoglycosides (particularly gentamicin), ansamycins, carbacephem, carbapenems, cephalosporins (particularly cephalosporin of first, second, third generation), glycopeptides, glycylcyclines, lipiarmycins (such as fidaxomicin), lincosamides, lipopeptide, macrolides, monobactams, nitrofurans, oxazolidonones (such as linezolid), penicillins, penicillin combinations, polymixins, polypeptides, rifamycins, quinolones, sulfonamides, tetracyclines, drugs against mycobacteria. Specific examples of such substances include: amikacin, gentamicin, silver, colloidal silver, silver powder, silver nanoparticles, silver compounds, silver releasing compounds, kanamycin, neomycin, netilmicin, tobramycin, paromomycin, spectinomycin, geldanamycin, herbimycin, 'rifaximin', streptomycin, loracarbef, ertapenem, doripenem, 'imipenem'/cilastatin, meropenem, cefadroxil, cefazolin, 'cefalotin' or cefalothin, cefalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, clindamycin, lincomycin, daptomycin, azithromycin, clarithromycin, dirithromycin, erythromycin, roxithromycin, troleandomycin, telithromycin, spiramycin, aztreonam, furazolidone, nitrofurantoin, linezolid, posizolid, radezolid, torezolid, amoxicillin, ampicillin, azlocillin, carbenicillin, cloxacillin, dicloxacillin, flucloxacillin, mezlocillin, methicillin, nafcillin, oxacillin, penicillin G, penicillin V, piperacillin, temocillin, ticarcillin, amoxicillin/clavulanate, ampicillin/sulbactam, piperacillin/tazobactam, ticarcillin/clavulanate, bacitracin, colistin, polymyxin B, ciprofloxacin, enoxacin, gatifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfacetamide, sulfadiazine, silver sulfadiazine, sulfadimethoxine, sulfamethizole, sulfamethoxazole, 'sulfanilimide' (archaic), sulfasalazine, sulfisoxazole 'trimethoprim'-sulfamethoxazole (co-trimoxazole) (TMP-SMX), sulfonamidochrysoidine (archaic), demeclocycline, doxycycline, minocycline, oxytetracycline, tetracycline, clofazimine, dapsone, capreomycin, cycloserine, ethambutol, ethionamide, isoniazid, pyrazinamide, 'rifampicin' (rifampin in US), rifabutin, rifapentine, streptomycin, arsphenamine, chloramphenicol, fosfomycin, fusidic acid, metronidazole, mupirocin, platensimycin, quinupristin/dalfopristin, thiamphenicol, tigecycline, tinidazole, trimethoprim, growth factors, enzymes, antitumoral drugs, anti-inflammatory drugs, antiviral drugs, antifungal drugs, anesthetics, anti-neoplastic drugs, antimitotic drugs, analgesics, narcotics, antithrombotic drugs, anticoagulants, haemostatic drugs.

More specifically the at least one bioactive agent may be selected from the group consisting of (a) proteins or (poly) peptides, such as erythropoietin (EPO), interferon-alpha, interferon-beta, interferon- gamma, growth hormone (human, pig, cow, etc.), growth factors such as transforming growth factor-beta (TGF-beta), fibroblast growth factor (bFGF), vascular endothelial growth factor (VEGF), and the like, bone morphogenetic proteins (BMPs), BMP2, BMP7, OP1, dexamethasone and analogues, corticosteroids, fibronectin, fibrinogen, thrombin, proteins, GDF5, SDF1, CCL5, homing factors, TGS6, growth hormone releasing factor, nerve growth factor (NGF), granulocyte-colony stimulating factor (G-CSF), granulocyte macrophage-colony stimulating factor (GM-CSF), macrophage-colony stimulating factor (M-CSF), blood clotting factor, insulin, oxytocin, vasopressin, adrenocorticotropic hormone, epidermal growth factor, platelet-derived growth factor (PDGF), prolactin, luliberin, luteinizing hormone releasing hormone (LHRH), LHRH agonists, LHRH antagonists, somatostatin, glucagon, interleukin-1 (IL-1), interleukin-1 receptor antagonist (IL-1RA), interleukin-2 (IL-2), interleukin-11 (IL-11), gastrin, tetragastrin, pentagastrin, urogastrone, secretin, calcitonin, enkephalins, endorphins, angiotensins, thyrotropin releasing hormone (TRH) 5 tumor necrosis factor (TNF) 5 tumor necrosis factor related apoptosis inducing ligand (TRAIL) 5 heparinase, human atrial natriuretic peptide (hANP), glucagon-like peptide (GLP-I) 5 renin, bradykinin, bacitracins, polymyxins, colistins, tyrocidine, gramicidins, cyclosporins and synthetic analogs thereof, antibodies, monoclonal antibodies, polyclonal antibodies, and cytokines; and (b) vaccines; and (c) nucleic acid such as small interference RNA (siRNA), plasmid DNA, and antisense oligodeoxynucleotide (AS- ODN), viral vectors including adenoviruses, adenoassociated viruses, integrating viral vectors such as for example retroviruses, lentiviruses, non-viral vectors such as liposomes, charged polymers, inorganic salts capable of binding to genetic materials such as calcium phosphates ; and (d) hormones, such as testosterone, estradiol, progesterone, prostaglandins and synthetic analogs thereof; and (e) an anti-cancer drug, such as paclitaxel, doxorubicin, 5-fluorouracil, cisplatin, carboplatin, oxaliplatin, tegafur, irinotecan, docetaxel, cyclophosphamide, gemcitabine, ifosfamide, mitomycin C, vincristine, etoposide, methotrexate, topotecan, tamoxifen, vinorelbine, camptothecin, danuorubicin, chlorambucil, bryostatin-1, calicheamicin, mayatansme, levamisole, DNA recombinant interferon alfa-2a, mitoxantrone, nimustine, interferon alfa-2a, doxifluridine, formestane, leuprolide acetate, megestrol acetate, carmofur, teniposide, bleomycin, carmustine, heptaplatin, exemestane, anastrozole, estramustine, capecitabine, goserelin acetate, polysaccharide potassium, medroxypogesterone acetate, epirubicin, letrozole, pirarubicin, topotecan, altretamine, toremifene citrate, BCNU, taxotere, actinomycin D, polyethylene glycol conjugated protein, and synthetic analogs thereof; and (f) an angiogenesis inhibitor, such as Clodronate, Doxycycline, Marimastat, 2-Methoxyestradiol, Squalamine, Thalidomide,Combretastatin A4, Soy Isoflavone, Enzastaurin, CC 5013 (Revimid; Celgene Corp, Warren, NJ), Celecoxib, Halofuginone hydrobromide, interferon-alpha, Bevacizumab, Interleukin-12, VEFG-trap, Cetuximab, and synthetic analogs thereof.

In a further embodiment, the one or more bioactive agent may also be a (therapeutic) cell , preferably an autologous (therapeutic) cell, and may be selected from the group consisting of stem cells, mesenchymal stem cells, precondrocytes, nucleus pulposus cells preosteoblast, chondrocyte, umbilical vein endothelial cell (UVEC), osteoblast, adult stem cell, Schwann cell, oligodendrocyte, hepatocyte, mural cell (used in combination with UVEC), myoblast, insulin-secreting cell, endothelial cell, smooth muscle cell, fibroblast, [beta] -cell, endodermal cell, hepatic stem cell, juxraglomerular cell, skeletal muscle cell, keratinocyte, melanocyte, langerhans cell, merkel cell, dermal fibroblast, and preadipocyte.

In one embodiment the one or more bioactive agent may be present in an amount of between about 10ppm to 50 wt%, preferably about 50ppm to 25 wt% based on the total dry weight of the bioactive agent delivery system.

The bioactive agent may be dispersed within bioactive agent delivery system according to the present invention. Alternatively, the bioactive agent may be covalently linked to the copolymer composition of the invention though an amidation reaction between the bioactive agent and the DMTMM activated hyaluronic acid carboxyl moiety.

The bioactive agent delivery system may further comprise one or more additives dissolved in an aqueous solvent. These additives may include, but are not limited to, cationic polymers; anionic polymers; sugars; polyols, sugar-containing polyols and polymer-containing polyols, amino acids and sugar-containing amino acids, other bioavailable materials, sugar-containing ions, surfactants, organic solvents, preservatives, etc. More specifically the at least one additive may be selected from the group consisting of: (a) cationic polymers having the molecular weight from 200 to 750,000), such as, poly-L-arginine, poly-L-lysine, poly (ethylene glycol), polyethylenimine, chitosan, protamin, and the like; (b) neutral or anionic polymers such as poly (N-vinyl-2-pyrrolidone), polyvinylacetate (PVA), alginate, pristine hyaluronic acid and the like; (c) bioavailable materials such as amiloride, procainamide, acetyl-beta- methylcholine, spermine, spermidine, lysozyme, fibroin, albumin, collagen, growth factors such as transforming growth factor-beta (TGF-beta), fibroblast growth factor (bFGF), vascular endothelial growth factor (VEGF), and the like, bone morphogenetic proteins (BMPs), BMP2, BMP7, OP1, dexamethasone, fibronectin, fibrinogen, thrombin, proteins, dexrazoxane, leucovorin, ricinoleic acid, phospholipid, small intestinal submucosa, vitamin E, polyglycerol ester of fatty acid, Labrafil, Labrafil Ml 944CS, citric acid, glutamic acid, hydroxypropyl methylcellulose, gelatin, isopropyl myristate, Eudragit, tego betain, dimyristoylphosphatidylcholine, scleroglucan, and the like; (d) sugars, such as, starch, cyclodextrin and derivatives thereof, lactose, glucose, dextran, mannose, sucrose, trehalose, maltose, ficoll, and the like; (e) polyols, such as, innositol, mannitol, sorbitol, and the like, sugar-containing polyols, such as, sucrose-mannitol, glucose-mannitol, and the like, and polymer-containing polyols, such as, trehalose-PEG, sucrose-PEG, sucrose-dextran, and the like; (f) amino acids (including sugar-containing amino acids), such as, alanine, arginine, glycine, sorbitol-glycine, sucrose-glycine, peptide amphiphiles and the like,- (g) sugar-containing ions, such as, trehalose-ZnS04, maltose-ZnSO4, and the like; and bioacceptable salts, such as, silicate, NaCl, KCl, NaBr, NaI, LiCl, n-BvuNBr, n-Pr₄NBr, Et₄NBr, Mg(OH)₂, Ca(OH)_{2,}ZnCO₃, Ca₃(PO4)₂, ZnCl₂, (C₂H₃O₂)₂Zn, ZnCO₃, CdCl₂, HgCl₂, CoCl₂, (CaNOs)₂, BaCl₂, MgCl₂, PbCl₂, AlCl₃, FeCl₂, FeCl₃, NiCl₂, AgCl, AuCl₃, CuCl₂, sodium tetradecyl sulfate, dodecyltrimethylammonium bromide, dodecyltrmethylammonium chloride, tetradecyltrimethylammonium bromide, and the like; organic solvents, such as, cremophor EL, ethanol, dimethyl sulfoxide, and the like; (h) preservatives, such as, methylparaben and the like; and (i) surfactants, such as, poloxamer of various molecular weights, tween 20, tween 80, triton X-IOO, sodium dodecyl sulfate (SDS, Brij) and the like.

The one or more additive may be added to the bioactive agent delivery system by incorporation below the LCST, e.g. by dispersion, and may be present from 10ppm by weight to 50 wt%, preferably about 50ppm by weight to 25 wt%, based on the total dry weight of the bioactive agent delivery system.

In another specific embodiment one or more bioactive agent and optionally an additive are added to a water dispersion of the copolymer of the invention to form the bioactive agent delivery system, which may be used immediately.

In a further aspect of the invention the bioactive agent delivery system may further comprise inorganic salts or ceramic materials in up to 80 wt%, preferably of inorganic salts, minerals or ceramic materials known in the art as osteoconductive materials, and more preferably up to 80 wt% of biphasic calcium phosphate, the weight percent being based on the total weight of the bioactive agent delivery system comprising the inorganic salt or ceramic material. Biphasic calcium phosphate comprises, and preferably consists of, a mixture of hydroxyapatite and beta tricalcium phosphate.

In a further aspect the invention is directed towards further uses of the thermosensitive hyaluronic acid conjugates, such as for drug delivery, infection prevention, infection eradication, adhesion prevention with special emphasis on abdominal, pelvic and spine surgery adhesion prevention, viscosupplementation, in vitro cell culture, tissue filler and augmentation, cartilage regeneration, intervertebral disc regeneration, bone healing and regeneration, spinal fusion and tissue engineering applications in cosmetic, plastic and aesthetic surgery, regenerative medicine, ophthalmology, ophthalmic surgery, general surgery, rheumatology, orthopedics and trauma.

Thus in another embodiment, bioactive agents covalently linked to or dispersed in thermosensitive hyaluronic acid conjugates may be used to investigate the impact of said bioactive agents on cells cultured in vitro.

In another embodiment, the thermosensitive hyaluronic acid conjugates of the invention may be used as a cell culture device. Cells may be suspended in the liquid solution of conjugate at room temperature and then warmed to create a robust, stable hydrocolloid gel for three dimensional cell culture. Retrieval of the cells may be achieved by simply cooling the hydrocolloid gel such that it returns to its liquid solution state.

Due to its thermosensitive nature the thermosensitive hyaluronic acid conjugates or bioactive agent delivery systems of the invention may be administered to a subject through various routes depending on its final use.

These routes of administration may include oral administration, buccal administration, mucosal administration, nasal administration, intraperitoneal administration, hypodermic injection, muscular injection, intraarticular injection, intradiscal administration, spine administration, percutaneous administration, topical administration and intratumoral administration, whereby a local administration such as hypodermic injection, intraarticular administration, topical administration, muscular injection, or percutaneous administration is preferred.

In one embodiment, the bioactive agent delivery system may be injected as an aqueous solution into a living body (at a temperature below the LCST). Upon injection the bioactive agent delivery system forms a bioactive agent-containing depot in a stiff hydrocolloid gel state at in vivo temperature. Release (or diffusion) of the bioactive agent occurs upon degradation of the copolymer composition or via simple diffusion from the depot. In case of a chemically incorporated bioactive agent (i.e. by covalent linkage) the release or diffusion also depends on the degradation of the chemical linkage to the polysaccharide backbone.

In another embodiment, the bioactive agent delivery system may be used topically in the form of dry spray, wet spray, foam, cream, ointment, lotion, emulsion.

In another specific embodiment the bioactive delivery system may be used for the topical treatment of wounds for infection prevention, infection management, antiseptic agent, bacteriostatic agent.

Another specific embodiment is the use of the hyaluronic acid conjugate of the present invention in combination with pharmaceutical ingredients, additives, excipients for the preparation of topical formulations aimed at delivering antibiotics, antimicrobials, disinfectants, antiseptic, bactericidal and bacteriostatic substances for immediate treatment of injuries, lacerations, cuts, wounds involving soft and/or hard tissues before the transport of the patient to a suitable care unit.

Prior to its use as a bioactive agent delivery system, the hyaluronic acid conjugate may be stored in lyophilized form until further use. Thus in a further aspect, the invention provides a single or multi compartment kit comprising a hyaluronic acid conjugate composition according to the invention in a sterile, lyophilized form. In a specific embodiment, the kit may comprise in separate compartments a defined amount of aqueous solvent for reconstitution of the conjugate composition and/or a defined amount of a bioactive agent and/or a defined amount of an additive.

### EXPERIMENTS

The invention is now described in non-limiting, greater detail in the following examples, for the purpose, of illustrating possible embodiments thereof, which can in any case be varied by experts in the field.

### Example 1: Synthesis of HA-pNIPAM conjugate 280-45-6.5

1.08g of the sodium salt of hyaluronic acid (HA) of average molecular weight 280 kDa are solubilized in 60 ml of water. 3.5g of amino-terminated poly(N-isopropylacrylamide) (pNIPAM) of average molecular weight 45 kDa are dissolved in 40 ml of water. Solutions are combined and let cool down at + 4°C, and 780mg of DMTMM in powder are added. The reaction mixture is left to react at + 4°C for 5 days, after which the solution is warmed up at 50°C to obtain a jelly mass which is washed thoroughly with boiling pure water until by-products elimination. The washed product is collected and freeze dried to constant weight. Characterization via NMR reveals a molar degree of substitution of 6.5%.

### Example 2: Synthesis of HA-pNIPAM conjugate 280-25-9

1.08g of the sodium salt of HA of average molecular weight 280 kDa are solubilized in 60 ml of water. 3.5g of amino-terminated pNIPAM of average molecular weight 25 kDa are dissolved in 40 ml of water. Solutions are let cool down at + 4°C and combined, and 780mg of DMTMM in powder are added. The reaction mixture is let to react at + 4°C for 5 days, and freeze-dried to constant weight after exhaustive dialysis. Characterization via NMR reveals a molar degree of substitution of 9%.

### Example 3: Synthesis of HA-pNIPAM conjugate 280-45-5

1.08g of the sodium salt of HA average molecular weight 280 kDa are solubilized in 60 ml of water. 2.5g of amino-terminated pNIPAM average molecular weight 45 kDa are dissolved in 40 ml of water. Solutions are combined and let cool down at + 4°C, and 780mg of DMTMM in powder are added. The reaction mixture is let to react at + 4°C for 5 days, after which the solution is warmed up at 50°C to obtain a jelly mass which is washed thoroughly with boiling pure water until by-products elimination. The washed product is collected and freeze dried to constant weight. Characterization via NMR reveals a molar degree of substitution of 5%.

### Example 4: Synthesis of HA-pNIPAM conjugate 280-25-7

1.08g of the sodium salt of HA average molecular weight 280 kDa are solubilized in 60 ml of water. 2.5g of amino-terminated pNIPAM average molecular weight 25 kDa are dissolved in 40 ml of water. Solutions are combined and let cool down at + 4°C, and 780mg of DMTMM in powder are added. The reaction mixture is let to react at + 4°C for 5 days, after which the solution is warmed up at 50°C to obtain a jelly mass which is washed thoroughly with boiling pure water until by-products elimination. The washed product is collected and freeze dried to constant weight. Characterization via NMR reveals a molar degree of substitution of 7%.

### Example 5: Synthesis of HA-pNIPAM conjugate 1590-25-6

2.18 g of the sodium salt of HA average molecular weight 1590 kDa are solubilized in 170 ml of water. 5g of amino-terminated pNIPAM average molecular weight 25 kDa are dissolved in 80 ml of water. Solutions are combined and let cool down at + 4°C, and 1.5 g of DMTMM in powder are added. The reaction mixture is let to react at + 4°C for 5 days, after which the solution is warmed up at 50°C to obtain a jelly mass which is washed thoroughly with boiling pure water until by-products elimination. The washed product is collected and freeze dried to constant weight. Characterization via NMR reveals a molar degree of substitution of 6%.

### Example 6: Synthesis of HA-pNIPAM conjugate 1590-45-5

1.08g of the sodium salt of HA average molecular weight 280 kDa are dissolved in 60 ml of water. 2.5g of amino-terminated pNIPAM average molecular weight 45 kDa are dissolved in 40 ml of water. Solutions are combined and let cool down at + 4°C, and 780mg of DMTMM in powder are added. The reaction mixture is let to react at + 4°C for 5 days, after which the solution is warmed up at 50°C to obtain a jelly mass which is washed thoroughly with boiling pure water until by-products elimination. The washed product is collected and freeze dried to constant weight. Characterization via NMR reveals a molar degree of substitution of 5%.

### Example 7: Synthesis of HA-pNIPAM conjugate 650-45-5

1.08g of the sodium salt of HA average molecular weight 650 kDa are dissolved in 60 ml of water. 2.5g of amino-terminated pNIPAM average molecular weight 45 kDa are dissolved in 40 ml of water. Solutions are combined and let cool down at + 4°C, and 780mg of DMTMM in powder are added. The reaction mixture is let to react at + 4°C for 5 days, after which the solution dialyzed thoroughly against water until by-products elimination. The product is collected and freeze dried to constant weight. Characterization via NMR reveals a molar degree of substitution of 5%.

### Example 8: Synthesis of HA-Jeffamine M-600 conjugate

1.08g of the sodium salt of HA average molecular weight 280 kDa are solubilized in 60 ml of water. 0.8g of a polyetheramine having a PO/EO ratio of 9/1 and a molecular weight of 600 Da commercially available under the trademark JEFFAMINE® M-600 are dissolved in 40 ml of water. Solutions are combined and allowed to cool down to + 10°C, and 975 mg of DMTMM in powder are added. The reaction mixture is left to react at + 10°C for 3 days, after which the solution is added to NaCl to realize a concentration of 9g/l, precipitated with ethanol and washed with ethanol/water 4 / 1 until NaCl and by products elimination. The product is washed with absolute ethanol and dried until residual solvents elimination. Characterization via NMR reveals a molar degree of substitution of 28.7%.

### Example 9: Synthesis of HA-Jeffamine® M-2005 conjugate

2.15 g of the sodium salt of HA average molecular weight 650 kDa are solubilized in 120 ml of water. 2.5 g of a polyetheramine having a PO/EO ratio of 29/6 and a molecular weight of 2 kDa commercially available under the trademark JEFFAMINE® M-2005 are dissolved in 80 ml of water. Both solutions are cooled down to + 4°C, and combined with 2.22 g of DMTMM in powder. The reaction mixture is left to react at + 4°C for 5 days, after which the solution is dialyzed exhaustively against water until by-products elimination. The product is collected and freeze dried to constant weight. Characterization via NMR reveals a molar degree of substitution of 23%.

### Example 10: Synthesis of HA-pNIPAM conjugate according to the prior art (CDI-mediated conjugation)

HA-pNIPAM conjugate is synthesized using 1,1'-carbonyldiimidazole according to the procedure in Carbohydrate Polymers 90 (2012) 1378-1385.

HA of average molecular weight of 280 kDa and pNIPAM of average molecular weight of 45 kDa are used in order to reproduce the product obtained as of example 1 above.

Characterization via NMR reveals a molar degree of substitution of 6.5%. The product was analyzed for its rheological profile as a function of the temperature according to the procedure described in example 12.

### Example 11: Synthesis of HA-pNIPAM conjugate according to the prior art (EDC+NHS mediated conjugation)

HA-pNIPAM conjugate is synthesized using 1-ethyl-3-(3-dimethylaminopropyl)carbodiimide according to the procedure in Journal of Biomaterials and Nanobiotechnology, 3, 1-9. The product was analyzed for its rheological profile as a function of the temperature according to the procedure described in example 12.

### Example 12: Comparison of rheological profiles of HA-pNIPAM solutions prepared with different methods

A 10% w/v solution of thermosensitive hyaluronic acid conjugate is prepared by dissolving the dried conjugates obtained through the procedure of Example 1, 10, and 11 in phosphate buffer saline.

The viscoelastic shear moduli of the three thus prepared solutions is then measured between 20 and 40 °C using an Anton Paar MCR302 rheometer equipped with a Peltier loading cell at the frequency of 1 Hz and 1% strain.

The main performance indicators are represented in Table 1, namely viscosity at room temperature (Viscous Modulus) and the magnitude of the transition above and below LCST (ratio between shear storage modulus G'at 35 and 25°C).

**Table 1**

| Conjugate | Viscous Modulus at 25°C | G' (35°C) / G' (25°C) |
|---|---|---|
| of Example 1 | 18 Pa | 251 |
| of Example 10 | 0.38 Pa | 726 |
| of Example 11 | 98.6 Pa | 3.58 |

As can be seen from Table 1, the conjugate according to Example 11 using EDC/NHS displays only minor changes in viscoelasticity upon temperature increase as indicated by the ratio between G'(35°C)/G'(25°C), which precludes its use in biomedical applications. The conjugate according to Example 10 displays an important viscoelasticity change, but starts from an undesirably low viscosity at room temperature (25°C), which also precludes its use from biomedical applications.

In contrast, the conjugate according to Example 1 displays both good viscoelasticity and cohesiveness at room temperature (25°C) and an evident transition to the hydrocolloid state. For biomedical applications such as coating, the improved viscosity gives better cohesion; whereas for use as implantable material via cannula injection the improved viscosity helps to give better cohesion at the site of implantation and to avoids flow and the systemic spreading of the conjugate; and for use as drug delivery system the increased viscosity slows down the release of the drug, limiting the free diffusion.

### Example 13: Synthesis of HA-pNIPAM conjugate 150-39-7

1.08g of the sodium salt of HA average molecular weight 150 kDa are solubilized in 60 ml of water. 3.5g of amino-terminated pNIPAM average molecular weight 45 kDa are dissolved in 40 ml of water. Solutions are combined and left to cool down to + 4°C, and 780mg of DMTMM in powder are added. The reaction mixture is left to react at + 4°C for 5 days, after which the solution is warmed up at 50°C to obtain a jelly mass which is washed thoroughly with boiling pure water until by-products elimination. The washed product is collected and freeze dried to constant weight. Characterization via NMR reveals a molar degree of substitution of 7%.

### Example 14: Synthesis of HA-pNIPAM conjugate 150-45-9

1.08g of the sodium salt of HA average molecular weight 150 kDa are solubilized in 60 ml of water. 3.5g of amino-terminated pNIPAM average molecular weight 45 kDa are dissolved in 40 ml of water. Solutions are combined and let cool down at + 5°C, and 780mg of DMTMM in powder are added. The reaction mixture is left to react at + 5°C for 7 days, after which the solution is warmed up at 50°C to obtain a jelly mass which is washed thoroughly with boiling pure water until by-products elimination. The washed product is collected and freeze dried to constant weight. Characterization via NMR reveals a molar degree of substitution of 9%.

### Example 15: Synthesis of HA-pNIPAM-SDF1 graft conjugate

1.08g of the sodium salt of HA average molecular weight 150 kDa are solubilized in 60 ml of water. 3.5g of amino-terminated pNIPAM average molecular weight 45 kDa and 5 mg of stromal cell-derived factor 1 (SDF1) are dissolved in 40 ml of water. Solutions are combined and left to cool down to + 4°C, and 780mg of DMTMM in powder are added. The reaction mixture is let to react at + 4°C for 5 days, after which the solution is dialyzed at + 4°C against water until unbound molecules and by-products are eliminated. The purified product is collected and freeze dried to constant weight. Characterization via NMR reveals a molar degree of substitution of pNIPAM of 6.5%, while a chemoattraction assay confirms that grafted SDF1 maintains its biological activity, **confirming the suitability of the formulation as system for the attraction of stem cells in regenerative medicine.**

### Example 16: Synthesis of HA-pNIPAM-BMP2 graft conjugate

1.08g of the sodium salt of HA average molecular weight 150 kDa are solubilized in 60 ml of water. 3.5g of amino-terminated pNIPAM average molecular weight 45 kDa and 12 mg of bone morphogenetic protein 2 (BMP2) are dissolved in 40 ml of water. Solutions are combined and let cool down at + 4°C, and 780mg of DMTMM in powder are added. The reaction mixture is left to react at + 4°C for 5 days, after which the solution is dialyzed at + 4°C against water until unbound molecules and by-products are eliminated. The purified product is collected and freeze dried to constant weight. Characterization via NMR reveals a molar degree of substitution of pNIPAM 6.5%, while osteogenic potential was confirmed by alkaline phosphatase activity and mineralization assay.

### Example 17: Preparation of hydrocolloid composites with Calcium Phosphate for BMP2 release

HA-pNIPAM conjugate is prepared as of per example 1. 80mg of the conjugate are mixed with 250mg of biphasic calcium phosphate (a combination of hydroxyapatite and beta tricalcium phosphate) in granules until homogeneity. The composite material is then combined with a solution of 30 µg of BMP2 in phosphate buffer saline (PBS) in order to prepare 400µl of composition. The release of BMP2 from the composite is evaluated simulating physiological conditions with ELISA detection (supplier, R&D Systems). The concentration profile shows a controlled release over 20 days, confirming the suitability of the formulation as sustained release system for bone regeneration.

### Example 18: Preparation of a conjugate solution for CCL5 release

HA-pNIPAM conjugate is prepared as of per example 1. 50mg of the conjugate are combined with 420µl of PBS solution containing 4.7 µg of chemokine ligand 5 (CCL5). The release of the factor from the composite is evaluated simulating physiological conditions and using the ELISA method (supplier, R&D Systems). The concentration profile shows a controlled release over 14 days, confirming the suitability of the formulation as sustained release system for the attraction of stem cells in regenerative medicine.

### Example 19: Preparation of a conjugate solution for kartogenin delivery

HA-pNIPAM conjugate is prepared as of per example 1. 50mg of the conjugate are combined with 420µl of kartogenin solution (132nM) in PBS. The release of the factor from the composite is evaluated simulating physiological conditions over a period of 7 days. 20% of the amount of kartogenin is released during the first 24 hours, while a further 38% is released over the week of observation, confirming the suitability of the formulation as sustained release system for the attraction of stem cells in regenerative medicine and cartilage regeneration.

### Example 20: Preparation of a composite with Calcium Phosphate including Strontium ranelate

HA-pNIPAM conjugate is prepared as of per example 1. 80mg of the conjugate are mixed with 280mg of biphasic calcium phosphate (a combination of hydroxyapatite and beta tricalcium phosphate) in granules until homogeneity. The composite material is then combined with a solution of 30 µg of strontium ranelate in PBS in order to prepare 400µl of composition. The release of strontium ranelate from the composite is evaluated simulating physiological conditions over 4 weeks using atomic spectroscopy detection. The concentration profile shows a controlled release over 28 days.

### Example 21: Preparation of a conjugate solution for antibiotic release

HA-pNIPAM conjugate is prepared as of per example 1. 140mg of the conjugate are combined with a solution of 1.5 mg of gentamicin in PBS in order to prepare 1 ml of composition. The release of gentamicin from the composite is evaluated simulating physiological conditions over 4 weeks using HPLC detection and pre-column functionalization with phthaldialdehyde and fluorescent detection. The concentration profile shows a controlled release over 28 days.

### Example 22: Preparation of a foam formulation for antibiotic release

The gentamicin-releasing formulation prepared as per of example 21 is combined with suitable pharmaceutical excipients in order to produce a foamy formulation. The release of gentamicin from the formulation is evaluated simulating physiological conditions over 4 weeks using HPLC detection and pre-column functionalization with phthaldialdehyde and fluorescent detection. The concentration profile shows a controlled release over 28 days, confirming the suitability of the formulation for the infection prevention and management in superficial and deep wounds. The formulation is particularly suited for the immediate management of wounds and trauma.

### Example 23: Preparation of a conjugate solution for antibiotic release

The gentamicin-releasing formulation prepared as per of example 21 is combined with suitable pharmaceutical excipients in order to produce emulsion, lotion, cream, ointment formulations. The release of gentamicin from the composite is evaluated simulating physiological conditions over 4 weeks using HPLC detection and pre-column functionalization with phthaldialdehyde and fluorescent detection. The concentration profile shows a controlled release for over 23 days, confirming the suitability of the formulation for the infection prevention in superficial and deep wounds.

### Example 24: Preparation of a conjugate solution for cell delivery

HA-pNIPAM conjugate is prepared as of per example 1. 240 mg of the conjugate are dispersed until homogeneity in phosphate buffer saline in order to prepare 2 ml of composition. Four millions of Mesenchymal Stem Cells are homogeneously dispersed within the conjugate solution, which is flowable at room temperature but turns into a stiff hydrocolloid above 30°C. The system is suitable for in vitro cell culture as well as for the delivery of cells into living systems for regenerative medicine.

## Claims

1. A method for preparing a thermosensitive hyaluronic acid conjugate comprising the steps of contacting in any order, preferably in this order,
a. a predefined amount of hyaluronic acid,
b. a predefined amount of a 1,3,5-triazine (or s-triazine) compound, and
c. a predefined amount of one or more thermosensitive polymers having at least one terminal amine moiety.

2. The method according to claim 1, wherein the hyaluronic acid and the thermosensitive polymer are jointly solubilized in an aqueous solvent, and are preferably solubilized in an aqueous buffer solution having a pH of from 4.0 to 9.5 and/or wherein the aqueous solvent or aqueous buffer solution is essentially free of organic solvents.

3. The method according to any preceding claim wherein the thermosensitive polymer having at least one terminal amine moiety is chosen among poly(N-alkyl(meth)acrylamide)s such as poly(N-isoalkyl(meth)acrylamide), preferably poly(N-isopropylacrylamide), polyetheramines such as monoamine polyetheramine or diamine polyetheramine, preferably a monoamine polyetheramine, or poloxamers comprising a central chain of poly(propylene oxide) flanked by chains of poly(ethylene oxide), having at least one terminal amine moiety.

4. The method according to any preceding claim, wherein the hyaluronic acid has a molecular weight of from 50 to 10 000 kDa, preferably 50 to 2 500 kDa and more preferably of from 100 to 1000 kDa and/or the one or more thermosensitive polymers have a molecular weight of from 5 to 200 kDa, preferably of from 10 to 100 kDa.

5. The method according to any preceding claim, wherein the 1,3,5-triazine (or s-triazine) compound is a salt of 4-(4,6-dialkyloxy-1,3,5-triazin-2-yl)-4-methyl morpholinium and more preferably is 4-(4,6-dimethoxy-1,3,5-triazin-2-yl)-4-methyl morpholinium chloride or 4-(4,6-dimethoxy-1,3,5-triazin-2-yl)-4-methyl morpholinium tetrafluoroborate, and/or wherein the molar ratio between the available carboxylic acid moieties of hyaluronic acid and the 1,3,5-triazine (or s-triazine) compound is of from 0.05 to 20, preferably of from 0.5 to 10, more preferably from 0.8 to 4, and most preferably of from 1 to 2.

6. The method according to any preceding claims wherein it further comprises a step of d. isolating the hyaluronic acid conjugate by increasing the temperature above the lower critical solution temperature (LCST) of said hyaluronic acid conjugate.

7. The method according to any preceding claim, wherein said method, and preferably step c., further comprises a predetermined amount of one or more bioactive agents.

8. A thermosensitive hyaluronic acid conjugate obtained by the method of any preceding claim comprising a thermosensitive polymer conjugated via 1,3,5-mediated direct amidation.

9. The thermosensitive hyaluronic acid conjugate according to claim 8 having a degree of substitution of from 0.1 to 50%, preferably of from 2 to 30%, more preferably of from 2.5 to 25% when measured by nuclear magnetic resonance spectroscopy and/or wherein the thermosensitive polymer having at least one terminal amine moiety is a poly(N-isoalkyl(meth)acrylamide) having a molecular weight of from 10 to 100 kDa, preferably of from 20 to 50 kDa, more preferably of from 30 to 50 kDa.

10. A bioactive agent delivery system comprising the thermosensitive hyaluronic acid conjugate according to claim 8 to 9 and one or more bioactive agents,
wherein the bioactive agent is a protein or (poly)peptide, a vaccine, a nucleic acid optionally delivered through a gene vector, a hormone, a cancer drug, or an angiogenesis inhibitor, a growth factor or an anti-microbial substance; or a (therapeutic) cell, preferably an autologous (therapeutic) cell or an antibiotic, preferably gentamycin.

11. The bioactive agent delivery system of claim 10, wherein it further comprises an osteoconductive material in an amount of from 0.1 to 80 wt%, the weight percent being based on the total weight of the bioactive agent delivery system, and/or the osteoconductive material is chosen among inorganic salts, minerals or ceramic materials and wherein it preferably is biphasic calcium phosphate.

12. A solution of the thermosensitive hyaluronic acid conjugate according to claim 8 to 9 in an aqueous solvent or aqueous buffer solution, having a shear storage modulus G' in excess of 8 Pa below its lower critical solution temperature, preferably of from 8 to 100 Pa below its lower critical solution temperature and a ratio between said storage modulus G' above its lower critical solution temperature and below its lower critical solution temperature, i.e. G'(>LCST)/G'(<LCST), in excess of 80, preferably of from 80 to 8000, more preferably of from 80 to 4000, most preferably of from 100 to 3000.

13. A solution of the thermosensitive hyaluronic acid conjugate according to claim 8 to 9 in an aqueous solvent or aqueous buffer solution, having a shear storage modulus G' in excess of 8 Pa at 25°C, preferably of from 8 to 100 Pa at 25°C and a ratio between said storage modulus at 35°C and 25°C, i.e. G'(35°C)/G'(25°C), in excess of 80, preferably of from 80 to 8000, more preferably of from 80 to 4000, most preferably of from 100 to 3000.

## Patentansprüche

1. Ein Verfahren zur Herstellung eines thermosenisitven Hyaluronsäurekonjugats, umfassend die Schritte des Inkontaktbringens in beliebiger Reihenfolge, vorzugsweise in dieser Reihenfolge,
a. einer vordefinierten Menge an Hyaluronsäure,
b. einer vordefinierten Menge einer 1,3,5-Triazin-(oder s-Triazin)-Verbindung, und
c. einer vordefinierten Menge eines oder mehrerer thermosensitiver Polymere mit mindestens einer terminalen Amingruppe.

2. Das Verfahren gemäss Anspruch 1, wobei die Hyaluronsäure und das thermosensitive Polymer gemeinsam in einem wässrigen Lösungsmittel solubilisiert werden, und vorzugsweise in einer wässrigen Pufferlösung mit einem pH-Wert von 4.0 bis 9.5 solubilisiert werden und/oder wobei das wässrige Lösungsmittel oder die wässrige Pufferlösung im Wesentlichen frei von organischen Lösungsmitteln ist.

3. Das Verfahren gemäss irgendeinem vorhergehenden Anspruch, wobei das thermosensitive Polymer mit mindestens einer terminalen Amingruppe ausgewählt ist aus Poly(N-alkyl(meth)acrylamid)en wie Poly(N-isoalkyl(meth)acrylamid), vorzugsweise Poly(N-isopropylacrylamid), Polyetheramine wie Monoaminpolyetheramin oder Diaminpolyetheramin, vorzugsweise ein Monoaminpolyetheramin, oder Poloxamere, die eine zentrale Kette von Poly(propylenoxid) umfassen, die von Ketten aus Poly(ethylenoxid) flankiert ist, mit mindestens einer terminalen Amingruppe.

4. Das Verfahren gemäss irgendeinem vorhergehenden Anspruch, wobei die Hyaluronsäure ein Molekulargewicht von 50 bis 10 000 kDa, vorzugsweise 50 bis 2 500 kDa und besonders bevorzugt von 100 bis 1000 kDa und/oder der eine oder die mehreren thermosensitiven Polymere ein Molekulargewicht von 5 bis 200 kDa, vorzugsweise von 10 bis 100 kDa aufweisen.

5. Das Verfahren gemäss irgendeinem vorhergehenden Anspruch, wobei die 1,3,5-Triazin-(oder s-Triazin)-Verbindung ein Salz von 4-(4,6-Dialkyloxy-1,3,5-triazin-2-yl)-4-methylmorpholinium und besonders bevorzugt 4-(4,6-Dimethoxy-1,3,5-triazin-2-yl)-4-methylmorpholiniumchlorid oder 4-(4,6-Dimethoxy-1,3,5-triazin-2-yl)-4-methylmorpholiniumtetrafluoroborat ist, und/oder wobei das Molverhältnis zwischen den verfügbaren Karbonsäuregruppen der Hyaluronsäure und der 1,3,5-Triazin-(oder s-Triazin)-Verbindung 0.05 bis 20, vorzugsweise 0.5 bis 10, besonders bevorzugt 0.8 bis 4, und am meisten bevorzugt 1 bis 2, beträgt.

6. Das Verfahren gemäss irgendwelchen der vorhergehenden Ansprüche, wobei es ferner einen Schritt d., Isolieren des Hyaluronsäurekonjugats durch Erhöhung der Temperatur über die untere kritische Lösungstemperatur (LCST) des besagten Hyaluronsäurekonjugats, umfasst.

7. Das Verfahren gemäss einem der vorhergehenden Ansprüche, wobei das besagte Verfahren, und vorzugsweise Schritt c., ferner eine vorbestimmte Menge eines oder mehrerer bioaktiver Wirkstoffe umfasst.

8. Ein thermosensitives Hyaluronsäurekonjugat, erhalten durch das Verfahren nach irgendeinem vorhergehenden Anspruch umfassend ein thermosensitives Polymer konjugiert über 1, 3, 5-vermittelte direkte Amidierung.

9. Das thermosensitive Hyaluronsäurekonjugat gemäss Anspruch 8 mit einem Substitutionsgrad von 0.1 bis 50%, vorzugsweise von 2 bis 30%, besonders bevorzugt von 2.5 bis 25% bei Messung durch Kernspinresonanzspektroskopie und/oder wobei das thermosensitive Polymer mit mindestens einer terminalen Amingruppe ein Poly(N-isoalkyl(meth)acrylamid) mit einem Molekulargewicht von 10 bis 100 kDa, vorzugsweise von 20 bis 50 kDa, besonders bevorzugt von 30 bis 50 kDa, ist.

10. Ein bioaktives Wirkstoffabgabesystem, umfassend das thermosensitive Hyaluronsäurekonjugat gemäss Anspruch 8 bis 9 und ein oder mehrere bioaktive Wirkstoffe, wobei der bioaktive Wirkstoff ein Protein oder (Poly)peptid, ein Impfstoff, eine optionaler Weise durch ein Genvektor abgegebene Nukleinsäure, ein Hormon, ein Krebsmedikament, oder ein Angiogenese-Inhibitor, ein Wachstumsfaktor oder eine antimikrobielle Substanz ist; oder eine (therapeutische) Zelle, vorzugsweise eine autologe (therapeutische) Zelle oder ein Antibiotikum, vorzugsweise Gentamycin ist.

11. Das bioaktive Wirkstoffabgabesystem gemäss Anspruch 10, wobei es ferner ein osteokonduktives Material in einer Menge von 0.1 bis 80 Gew.-% umfasst, wobei die Gewichtsprozente auf dem Gesamtgewicht des bioaktiven Wirkstoffabgabesystems basiert, und/oder das osteokonduktive Material gewählt ist aus anorganischen Salzen, Mineralien oder keramischen Materialien und wobei es vorzugsweise zweiphasiges Kalziumphosphat ist.

12. Eine Lösung des thermosensitiven Hyaluronsäurekonjugats gemäss Anspruch 8 bis 9 in einem wässrigen Lösungsmittel oder einer wässrigen Pufferlösung mit einem Scherspeichermodul G' von mehr als 8 Pa unterhalb seiner unteren kritischen Lösungstemperatur, vorzugsweise von 8 bis 100 Pa unterhalb seiner unteren kritischen Lösungstemperatur und einem Verhältnis zwischen dem besagten Speichermodul G' oberhalb seiner unteren kritischen Lösungstemperatur und unterhalb seiner unteren kritischen Lösungstemperatur, d.h. G'(>LCST)/G'(<LCST), über 80, vorzugsweise von 80 bis 8000, besonders bevorzugt von 80 bis 4000, am meisten bevorzugt von 100 bis 3000.

13. Eine Lösung des thermosensitiven Hyaluronsäurekonjugats nach Anspruch 8 bis 9 in einem wässrigen Lösungsmittel oder einer wässrigen Pufferlösung mit einem Scherspeichermodul G' von mehr als 8 Pa bei 25°C, vorzugsweise von 8 bis 100 Pa bei 25°C und einem Verhältnis zwischen dem besagten Speichermodul bei 35°C und 25°C, d.h. G'(35°C)/G'(25°C), über 80, vorzugsweise von 80 bis 8000, besonders bevorzugt von 80 bis 4000, am meisten bevorzugt von 100 bis 3000.

## Revendications

1. Un procédé de préparation d'un conjugué thermosensible de l'acide hyaluronique qui comporte les étapes consistant à mettre en contact, dans n'importe quel ordre, préférablement dans cet ordre,
a. une quantité prédéfinie d'acide hyaluronique,
b. une quantité prédéfinie d'un composé de 1,3,5-triazine (ou s-triazine) ; et
c. une quantité prédéfinie d'un ou de plusieurs polymères thermosensibles ayant au moins une groupe amine terminal.

2. Le procédé selon la revendication 1, dans lequel l'acide hyaluronique et le polymère thermosensible sont solubilisés conjointement dans un solvant aqueux, et sont préférablement solubilisés dans une solution tampon aqueuse ayant un pH de 4.0 à 9.5 et / ou dans lequel le solvant aqueux ou la solution tampon aqueuse sont essentiellement libres de solvants organiques.

3. Le procédé selon l'une quelconque des revendications précédentes, dans lequel le polymère thermosensible ayant au moins un groupe amine terminal est choisi entre les poly(N-alkyle(méth)acrylamides) tel que le poly(N-isoalkyle(méth)acrylamide), préférablement le poly(N-isopropyleacrylamide), les polyétheramines tel que la monoaminpolyétheramine ou diaminepolyétheramine, préférablement une monoaminpolyétheramine, ou les poloxamères comprenant une chaîne centrale de poly(oxyde de propylène) flanquée de chaines de poly (oxyde d'éthylène), ayant au moins un groupe amine terminal.

4. Le procédé selon l'une quelconque des revendications précédentes, dans lequel l'acide hyaluronique a un poids moléculaire de 50 à 10 000 kDa, préférablement de 50 à 2 500 kDa et plus préférablement de 100 à 1 000 kDa et / ou l'un ou plusieurs polymères thermosensibles ont un poids moléculaire de 5 à 200 kDa, préférablement de 10 à 100 kDa.

5. Le procédé selon l'une quelconque des revendications précédentes, dans lequel le composé de 1, 3, 5-triazine (ou s-triazine) est un sel de 4-(4,6-dialkyleoxy-1,3,5-triazine-2-yle)-4-méthylemorpholinium et le plus préférablement la chlorure de 4- (4,6-diméthoxy-1,3,5-triazine-2-yle)-4-méthylemorpholinium ou le tetrafluoroborate de 4-(4,6-diméthoxy-1,3,5-triazine-2-yle)-4-methylemorpholinium, et / ou dans lequel le rapport molaire entre les groupes d'acide carboxylique disponibles de l'acide hyaluronique et le composé de 1,3,5-triazine (ou s-triazine) est de 0.05 à 20, préférablement de 0.5 à 10, plus préférablement de 0.8 à 4, et le plus préférablement de 1 à2.

6. Le procédé selon l'une quelconque des revendications précédentes, dans lequel celui-ci comprend en outre une étape d. de isoler le conjugué de l'acide hyaluronique en augmentant la température au-dessus de la température critique inférieure de solubilité (LCST) dudit conjugué de l'acide hyaluronique.

7. Le procédé selon l'une quelconque des revendications précédentes, dans lequel ledit procédé, et préférablement l'étape c., comprend en outre une quantité prédéfinie d'un ou plusieurs agents bioactifs.

8. Un conjugué thermosensible de l'acide hyaluronique obtenu par le procédé selon l'une quelconque des revendications précédentes, comprenant un polymère thermosensible conjugué à travers l'amidation directe véhiculée par la 1,3,5-triazine.

9. Le conjugué thermosensible de l'acide hyaluronique selon la revendication 8, ayant un degré de substitution de 0.1 à 50%, préférablement de 2 à 30%, plus préférablement de 2.5 à 25% lorsqu'il est mesuré par spectroscopie à résonance magnétique nucléaire et / ou dans lequel le polymère thermosensible ayant au moins un groupe amine terminal est un poly(N-isoalkyle (méth) acrylamide) ayant un poids moléculaire de 10 à 100 kDa, préférablement de 20 à 50 kDa, plus préférablement de 30 à 50 kDa.

10. Un système de distribution d'agent bioactif comprenant le conjugué thermosensible de l'acide hyaluronique selon la revendication 8 à 9 et un ou plusieurs agents bioactifs, dans lequel l'agent bioactif est une protéine ou un (poly)peptide, un vaccin, un acide nucléique optionnellement distribué à travers un vecteur génique, une hormone, un médicament contre le cancer ou un inhibiteur d'angiogenèse, un facteur de croissance ou une substance antimicrobienne ; ou une cellule (thérapeutique), préférablement une cellule autologue (thérapeutique) ou un antibiotique, préférablement la gentamycine.

11. Le système de distribution d'agent bioactif selon la revendication 10, dans lequel celui-ci comprend en outre un matériau ostéoconducteur dans une quantité de 0.1 à 80 pourcent en poids, les pourcents en poids étant basés sur le poids total du système de distribution d'agent bioactif, et / ou le matériau ostéoconducteur est choisi entre sels inorganiques, matières minérales ou céramiques et dans lequel il s'agit préférablement de phosphate de calcium diphasique.

12. Une solution du conjugué thermosensible de l'acide hyaluronique selon la revendication 8 à 9, dans un solvant aqueux ou une solution tampon aqueuse, ayant un module du cisaillement au stockage G' au-delà de 8 Pa en dessous de sa température critique inférieure de solubilité, préférablement de 8 à 100 Pa en dessous de sa température critique inférieure de solubilité, et un rapport entre ledit module au stockage G' au-dessus de sa température critique inférieure de solubilité et en dessous de sa température critique inférieure de solubilité est en dessous de sa température critique inférieure de solubilité c'est-à-dire G'(> LCST) / G' (<LCST), au-delà de 80, préférablement de 80 à 8000, plus préférablement de 80 à 4000, le plus préférablement de 100 à 3000.

13. Une solution du conjugué thermosensibles de l'acide hyaluronique selon la revendication 8 à 9, dans un solvant aqueux ou une solution tampon aqueuse, ayant un module du cisaillement au stockage G' au-delà de 8 Pa à 25 ° C, préférablement de 8 à 100 Pa à 25 ° C et un rapport entre ledit module au stockage à 35 ° C et 25 ° C, c'est-à-dire G'(35 ° C) / G' (25 ° C), au-delà de 80, préférablement de 80 à 8000, plus préférablement de 80 à 4000, le plus préférablement de 100 à 3000.
